# EUROPÄISCHE PATENTSCHRIFT

(11) **EP 0 792 128 B1**
(45) Veröffentlichungstag und Bekanntmachung des Hinweises auf die Patenterteilung: **12.04.2000**
(21) Anmeldenummer: 95936971.1
(22) Anmeldetag: 20.11.1995
(51) Int. Cl.: A61F 2/36, A61F 2/46

(54) **MODULARE HÜFTGELENKPROTHESE**
MODULAR ARTIFICIAL HIP JOINT
PROTHESE MODULAIRE D'ARTICULATION DE LA HANCHE

(30) Priorität: 19.11.1994 DE 4442205
(43) Veröffentlichungstag der Anmeldung: 03.09.1997
(73) Patentinhaber: MERCK PATENT GmbH, 64271 Darmstadt (DE)
(72) Erfinder: LOB, Günter, D-81377 München (DE); FISCHER, Hans-Joachim, D-12277 Berlin (DE); STEÜR, Gerd, D-10551 Berlin (DE); KRANZ, Curt, D-10825 Berlin (DE)
(74) Vertreter: Eisenführ, Speiser & Partner
(86) Internationale Anmeldenummer: DE9501653
(87) Internationale Veröffentlichungsnummer: WO9615738

(56) Entgegenhaltungen:
- EP-A- 0 135 755
- EP-A- 0 163 121
- EP-A- 0 190 981
- EP-A- 0 243 298
- EP-A- 0 359 457
- EP-A- 0 399 920
- EP-A- 0 498 518
- EP-A- 0 634 154
- DE-U- 9 418 963
- FR-A- 2 575 383
- FR-A- 2 629 707
- US-A- 5 002 578

## Beschreibung

Die Erfindung betrifft eine modulare Gelenkprothese der im Oberbegriff des Anspruchs 1 angegebenen Art, wie sie aus der EP-A-0 190 981 bekannt ist.

Derartige Gelenkprothesen werden in unterschiedlichsten Formen und Größen hergestellt, um eine möglichst genaue Anpassung an die jeweilige Knochenform und auch den aktuellen Zustand des Knochengewebes unter Berücksichtigung des Krankheitsbildes zu ermöglichen.

Durch mehrteilige Ausführung mit kraftschlüssiger Verbindung der entsprechenden Einzelteile im proximalen Bereich kann die Anpassung optimal erfolgen. Dabei ist gleichzeitig eine Positionierung des Gelenkkopfes unabhängig vom Schaftdurchmesser möglich.

Aus der EP-B1 0 243 298 ist ein Bausatz für eine Schaftprothese bekannt, der ein mit einer Gelenkkugel versehbares Kopfteil, ein im Knochen verankerbares Endteil und ein zwischen beiden positionierbares Zwischenteil aufweist. Alle Teile weisen konische Bohrungen bzw. dazu passende Zapfen auf, wodurch die Prothese durch Herstellen konischer Steckverbindungen zusammengesetzt werden kann. Kopf- und Zwischenteil weisen jeweils eine sich axiale Durchgangsbohrung auf.

Das Endteil ist mit Mitteln zum lösbaren Eingriff zwecks Übertragung einer Kraft in axialer Richtung versehen. Beim Zusammenfügen der Einzelteile sind die entsprechenden Bohrungen axial in Richtung des Schaftes ausgerichtet. Die Einzelteile der Prothese werden unter Verwendung eines eine Kraft in axialer Richtung übertragenden Zuganker zusammengefügt, welcher sowohl das Kopfteil als auch nachfolgende Schaftteile durchdringt und in die Gewindebohrung des Endteils einschraubar ist. Dadurch werden das Kopfteil, oder das Zwischenteil und das Endteil fest zusammengezogen, so daß eine Lockerung der einzelnen Prothesenteile durch die mechanische Belastung bei Benutzung nicht zu befürchten ist.

Die vorstehend beschriebene Lösung weist den Nachteil auf, daß bei einer derartigen Prothese die Steckverbindung zwischen den Einzelteilen im implantierten Zustand nicht gelöst werden kann, obgleich dies aus medizinischen Gründen - beispielsweise zum Nachsetzen oder Austausch des Kopfteils - gelegentlich erforderlich ist. Die konischen Steckverbindungen halten auch nach vorheriger Entfernung des im Schaftinneren vorgesehenen Zugankers derart fest zusammen, daß bei einer notwendigen Demontage zum Nachteil des Patienten Schädigungen des Femur oder zumindest eine unerwünschte Lockerung des Schafts unter Umständen nicht vermieden werden können.

Ausgehend von den Mängeln des Standes der Technik liegt der Erfindung die Aufgabe zugrunde, eine modularen Gelenkprothese der eingangs genannten Gattung zu schaffen, die auf einfache Weise ein Lösen der Einzelelemente untereinander auch von extern gestattet.

Diese Aufgabe wird mit den kennzeichnenden Merkmalen des Anspruchs 1 gelöst.

Die Erfindung schließt die Erkenntnis ein, daß mit einem Schraubbolzen erhebliche Druckkräfte in axialer Richtung erzeugt werden können, wenn sich das Bolzenende auf einem Widerlager abstützt. Hierzu ist auch der Rand der Bohrung eines nachfolgenden Schaftelements unter der Bedingung geeignet, daß der Außendurchmesser eines in die Gewindebohrung einzuschraubenden Bolzens größer ist als der Innendurchmesser des nachfolgenden Elements. Da ein Austausch (bei fortgeschrittenem Krankheitsbild oder aufgrund anderer Indikationen) vorzugsweise für das Kopfelement in Betracht kommt, können die nachfolgenden Elemente eine Bohrung mit konstantem Durchmesser aufweisen, wobei dann gegebenenfalls das (distal) letzte Element ein Gewinde (aufweist das einen Zuganker zum Verbinden der Schaftelemente bzw. zum Setzen der Konuselemente aufweist. Die Kraftwirkung zum Lösen zweier aufeinanderfolgender Elemente kann um so bequemer erreicht werden, je geringer seine Steigung gewählt ist.

Besonders vorteilhaft bei der Erfindung ist, daß ohne den im Knochenraum verbleiben unteren Teil des Schafts zu entfernen der gelenknahe Kopfteil entfernt und gegebenenfalls ersetzt werden kann. Damit ist eine wesentlich geringere Belastung des Patienten verbunden als bei herkömmlichen Prothesen. Außerdem kann bei entferntem Kopfteil der entsprechende Knocheninnenraum auch für andere Behandlungszwecke erreicht werden. Unter "Kopfteil" soll im vorliegenden Zusammenhang der gelenknahe Teil der Prothese verstanden werden, welche sich auch im Falle des Gelenkersatzes in anderen Körperregionen vorteilhaft verwenden läßt.

Als Modularsystem ausgebildete Prothesenschäfte weisen mindestens als Kopfteil und Endteil ein System verschiedener Einzelelemente unterschiedlicher Größe auf, welche vorzugsweise durch Zusammenstecken ihrer, entsprechende konische Zapfen oder Ausnehmung aufweisenden proximalen oder distalen Enden miteinander verbunden werden. Die erforderliche Stabilität der Steckverbindung wird durch - vorzugsweise als Zuganker ausgebildete - Schraubmittel gesichert. Das Endteil ist als Hohlschaft ausgebildet, wobei die sich axial erstreckende Längsbohrung des Trägerteils an ihrem proximalen Endabschnitt ein Gewinde trägt. Der Zuganker wird durch einen auf gleicher Achse wie die Längsbohrung im Trägerteil liegenden zylindrischen Kanal im Kopfteil geführt und in das mit einem Gewinde versehenen Endteil eingeschraubt.

Entsprechend der bevorzugten Ausführungsform der Erfindung ist der zylindrische Kanal im Kopfteil des modularen Hüftprothesenschaftes mindestens teilweise als Gewindebohrung ausgebildet. Diese Gewindebohrung weist dabei einen Durchmesser auf, welcher größer ist als der Durchmesser der den Zuganker aufnehmenden Gewindebohrung im nachfolgenden Schaftteil. Dieses Durchmessergestaltung sichert auf vorteilhafte Weise, daß bei zusammengesteckten Prothesenteilen auf dem proximal vorgesehenen, kegelstumpfförmigen Ende des nachfolgenden Schaftteils ein freier, im wesentlichen kreisringförmiger Flächenbereich der Deckfläche des Kegelstumpfes verbleibt. Dieser Flächenbereich steht als Widerlager für einen Schraubbolzen zur Verfügung, welcher nach Entfernen des Zugankers in das Kopfteil geschraubt werden kann. Der in das Kopfteil eingeschraubte Bolzen stützt sich auf dem im wesentlichen kreisringförmig ausgebildeten Widerlager ab. Bei einer weiteren Schraubbewegung wird in günstiger Weise eine axiale Druckkraft erzeugt, welche die (trotz fehlendem Zuganker) feste konische Steckverbindung zwischen Kopf- und nachfolgenden Schaftteil soweit löst, daß das Kopfteil zwecks Austausch entnommen oder bezüglich des nachfolgenden Schaftteils in gewünschtem Maße auf dessem am proximalen Ende vorgesehenen, kegelstumpfförmigen Zapfen geschwenkt werden kann. Um eine zur Kraftübertragung beim Lösen der Verbindung zwischen Kopf- und nachfolgenden Schaftteil ausreichend große Widerlagerfläche zu sichern, ist ein Durchmesserverhältnis der Bohrungen im Kopf- bzw. nachfolgenden Schaftteil im Bereich von 1,5 bis 2,5 günstig.

Nach einer anderen vorteilhaften Weiterbildung der Erfindung erstreckt sich das für den Schraubbolzen vorgesehene Gewinde im Kopfteil des modularen Hüftprothesenschaftes über die gesamte Länge des vorhandenen zylindrischen Kanals. Sowohl für die Gewindebohrung im Kopfteil des Hüftprothesenschaftes als auch für den Gewindeabschnitt am proximalen Ende des nachfolgenden Schaftteils ist ein metrisches Gewinde vorgesehen. Dabei weist vorzugsweise das Gewinde im Kopfteil eine geringere Steigung auf als das Gewinde am proximalen Ende des nachfolgenden Schaftteils und erleichtert dadurch das Erzeugen der zum Lösen der festen konischen Steckverbinduung erforderlichen Druckkraft durch den Schraubbolzen.

Gemäß einer weiteren vorteilhaften Ausführung der Erfindung können die beschriebenen Maßnahmen unter Benutzung verschiedener Abstufungen auch mehrfach bei aufeinanderfolgenden Elementen ein und desselben modularen Schafts verwendet werden. Bei der hier erfolgten Darstellung wäre hierbei lediglich das Wort "Kopfteil" durch "in Richtung nach distal vorangehendes Schaftelement" zu ersetzen. Es ist ersichtlich, daß die Stufungen der Durchmesser der Bohrungen sich dabei jeweils nach distal hin vergrößern.

Entsprechend einer zusätzlichen Weiterbildung der Erfindung weist das proximale Ende der im nachfolgenden Schaftteil vorgesehenen Gewindebohrung eine Ausnehmung auf, welche in günstiger Weise als symmetrisch zur Längsachse der Bohrung angeordnete Anfasung ausgebildet ist. Dadurch wird in vorteilhafter Weise erreicht, daß der Schraubbolzen, dessen freies Ende ebenfalls mit gleichem Winkel gefast ausgebildet ist, beim Abstützen auf dem proximalen Ende des Tragteils nicht mit dem Gewinde der in diesem Bereich vorgesehenen Gewindebohrung in Wirkkontakt gelangen kann und beim Erzeugen der zum Lösen der konischen Steckverbindung zwischen Kopf- und nachfolgenden Schaftteil erforderlichen Druckkraft eine Deformation der ersten Gewindegänge vermieden wird. Ein deformiertes Gewinde würde ein erneutes Verschrauben des Zugankers in das proximale Ende des nachfolgenden Schaftteils nach Anpassen oder Austausch des Kopfteils unmöglich machen. Für die Krafteinleitung in das Widerlager ist ein Neigungswinkel der Anfasung im Bereich von 45° in bezug auf die Mittelachse der entsprechenden Gewindebohrung günstig.

Das Kopfteil weist an seiner medialen Seite unterhalb des Konus für den Gelenkanschluß eine Auskehlung auf, welche in günstiger Weise einen Ansatz- und Auflagebereich für ein Werkzeug bildet, das zum Schwenken oder Abziehen des Kopfteils eingesetzt wird, nachdem die Konusverbindung zwischen Kopf- und nachfolgenden Schaftteil, wie vorstehend beschrieben, gelöst worden ist.

Um eine weitestgehende Anpassung des Gelenkschaftes an die anatomischen Bedingungen eines Patienten zu sichern, ist zur Herstellung eines individuell angepaßten Schaftes ein Bausatz mit unterschiedlich ausgebildeten Kopf- und nachfolgenden Schaftteilen von Vorteil. Die Einzelteile des Hüftgelenkschaftes unterscheiden sich in Durchmesser, Längenmaß und Krümmung. Sie sind unter Vorausetzung eines abgestimmten Außendurchmessers miteinander kombinierbar.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 die bevorzugte Ausführungsform der Erfindung in schematisierter Teilschnittdarstellung,
Figur 1a die Schnittansicht längs der Linie A...A gemäß Figur 1,

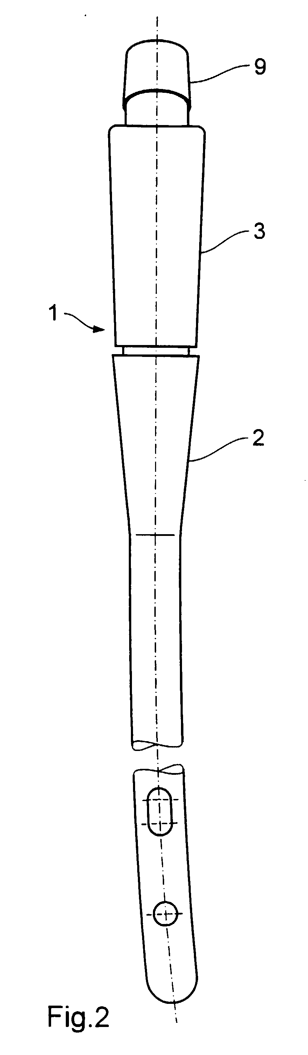

Figur 2 eine vereinfachte Darstellung der bevorzugten Ausführungsform der Erfindung gemäß Figur 1 als Seitenansicht von links sowie
Figur 3 eine vergrößerte Darstellung der Einzelheit E in Figur 1.

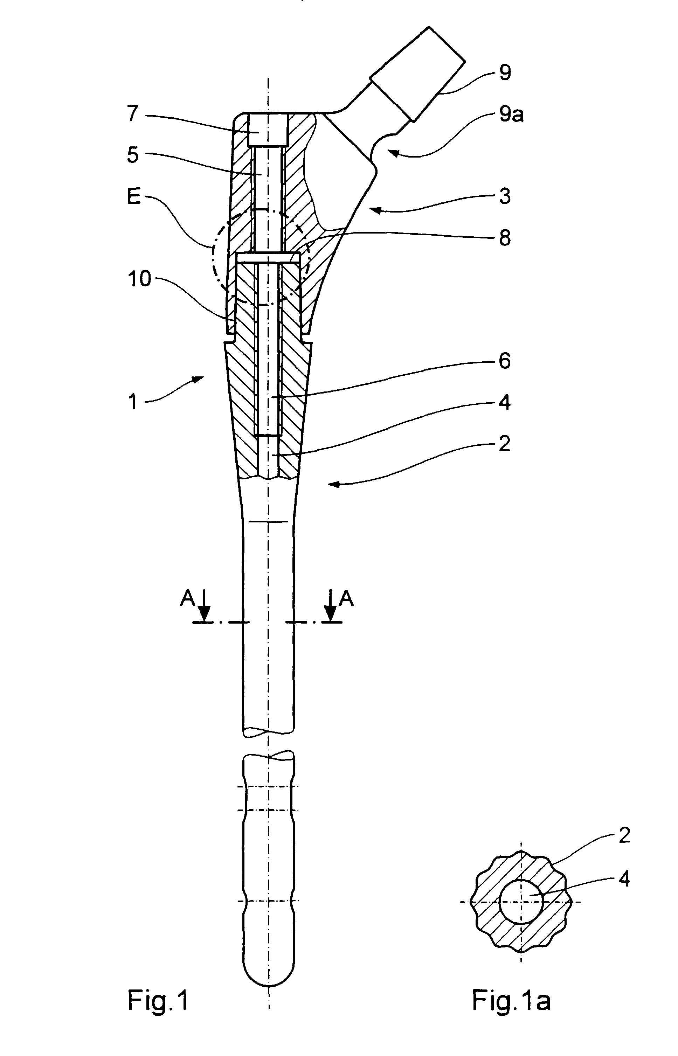

Der in den Figuren 1, la und 2 in Form einer Seitenansicht, eines Schnittes bzw. eines Teilschnittes dargestellten modularen Gelenkprothese 1 besteht aus einem Schaftteil 2 und einem Kopfteil 3, welche jeweils eine Profilierung in Längsrichtung aufweisen. Die Einzelteile 2 und 3 des Schaftes 1 sind mittels einer konischen Steckverbindung 10 auf gleicher Achse liegend und gegeneinander verschwenkbar verbunden. Für das "Setzen" des Konus und zur Sicherung dieser Steckverbindung gegen axiales Lösen ist ein (nicht dargestellter) Zuganker vorgesehen, welcher durch den als Gewindebohrung ausgebildeten Kanal 5 des Kopfteils 3 geführt und in den als Gewindebohrung 6 ausgebildeten proximalen Bereich des im nachfolgenden Schaftteil 2 vorgesehenen Kanals 4 geschraubt wird. Das Einschrauben erfolgt soweit, daß sich das proximale Ende des Zugankers in der Ausnehmung 7 abstützt und dabei die Einzelteile 2 und 3 des modularen Hüftprothesenschaftes 1 axial gegeneinander bewegt werden, bis die konische Steckverbindung 10 die gewünschte Festigkeit aufweist.

Um die Position des einen Gelenkanschluß-Konus 9 aufweisenden Kopfteil 3 in Bezug auf das nachfolgenden Schaftteil 2 ändern zu können oder das Kopfteil 3 auszutauschen, muß die konische Steckverbindung 10 erneut gelöst werden. Nach Lösen und Herausdrehen des (nicht dargestellten) Zugankers wird ein (ebenfalls nicht dargestellter) Schraubbolzen in die Gewindebohrung 5 des Kopfteils 3 eingeschraubt. Da der Durchmesser D₁ der Gewindebohrung 5 im Kopfteil 3 einen größeren Wert aufweist als der Durchmesser D₂ des proximalen, mit einem Gewinde versehenen Abschnitt 6 der zentralen Bohrung 4 des nachfolgenden Schaftteils 2, stützt sich der Schraubbolzen auf einem sich in seinem Flächenmaß durch die Durchmesserdifferenz D₁ - D₂ festgelegten Kreisring auf der Deckfläche 8 des als Kegelstumpf ausgebildeten proximalen Endes des Kopfteils 3 und ist somit in der Lage, eine axial gerichtete Druckkraft zu erzeugen. Diese Kraft löst die nach dem "Setzen" des Konus sehr feste Steckverbindung 10 und ermöglicht auf bequeme Art und Weise eine erneute Anpassung des Hüftprothesenschaftes 1 an veränderte körperliche Bedingungen eines Patienten.

Die an der medialen Seite des Kopfteils 3 unterhalb des Gelenkanschluß-Konus 9 vorgesehene Auskehlung 9a dient als Ansatzpunkt für ein Werkzeug, mit welchem das Kopfteil 3 nach erneutem Lösen der konischen Steckverbindung 10 zwischen Kopf- und nachfolgenden Schaftteil auf gleicher Achse geschwenkt oder im Bedarfsfall von dem nachfolgenden Schaftteil abgezogen werden kann.

In diesem Zusammenhang hat es sich für die Handhabbarkeit der Mittel zum Erzeugen der zum Lösen der konischen Steckverbindung erforderlichen Druckkraft als günstig erwiesen, ein Durchmesser-Verhältnis der Bohrungen 4, 5 im Bereich von 1,5 bis 2,5 vorzusehen und die Bohrung 5 über die gesamte Länge mit einem Gewinde zu versehen. Es werden bevorzugt metrische Gewinde verwendet, wobei das Gewinde der Gewindebohrung 5 im Kopfteil 3 eine geringere Steigung aufweist als das Gewinde im proximalen Abschnitt 6 der Bohrung 4 des nachfolgenden Schaftteils 2.

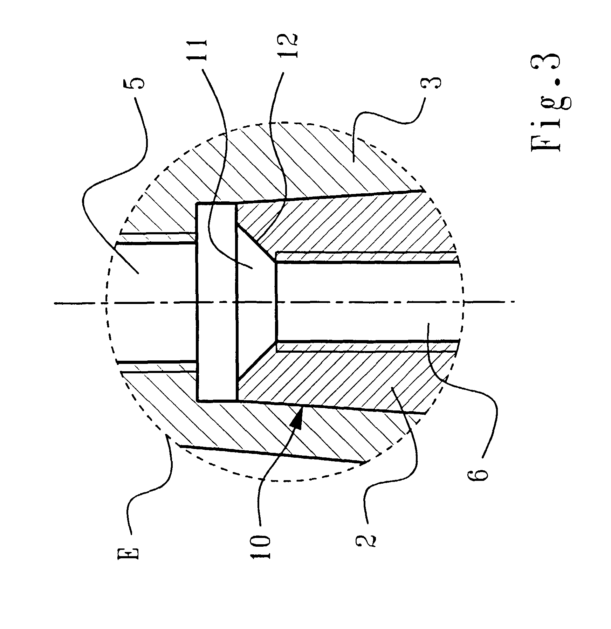

Die in Figur 3 vergrößert dargestellte Einzelheit E gemäß Figur 1 zeigt eine vorteilhafte Weiterbildung der in den Figuren 1, la und 2 gezeigten Erfindung. Um zu verhindern, daß durch den Wirkungseingriff des (nicht dargestellten) Schraubbolzens an der Oberseite des proximalen Endes des nachfolgenden Schaftteils 2 eine Beschädigung des dort vorgesehenen Gewindeabschnitts 6 möglich wird, ist an dem proximalen Ende der Gewindebohrung eine als Anfasung ausgebildete Ausnehmung 11 vorgesehen. Die Neigung der Flanken 12 beträgt 45° und entspricht der Flankenneigung der Fasung am Gewindeende des (nicht dargestellten) Schraubbolzens. Dadurch kann ein optimaler Krafteintrag in das nachfolgende Schaftteil 2 erfolgen, um das Lösen der konischen Steckverbindung 10 zwischen nachfolgenden Schaftteil 3 und Kopfteil 2 mit relativ geringem Krafteinsatz zu ermöglichen. Auf diese Weise läßt sich das Kopfteil bei implantiertem Schaft bei einer möglicherweise erforderlichen Nachoperation ohne wesentlichen Kraftaufwand und bei nur geringen Reaktionskräften entfernen. Damit wird ein Lösen bzw. Auslockern des im Knochen verbleibenden Schaftteils mit großer Sicherheit vermieden.

Es ist ersichtlich, daß - bei in der Zeichnung nicht dargestellten - weiteren Ausführungsformen - durch die erzielte Modularität in einem System von Schaftprothesen zur Erreichung unterschiedlicher individuell bemessener Prothesenformen verschiedene Elementegrößen kombinierbar sind. Die erfindungsgemäße Stufung der Gewindedurchmesser kann ebenfalls zwischen jeweils unterschiedlichen der aufeinanderfolgenden Elementen vorgesehen sein. Damit ist es beispielsweise möglich eine Trennung - wenn gewünscht - nicht nur zwischen dem Kopfteil und dem in Richtung distal nachfolgenden Schaftteil, sondern auch zwischen Schaftteilen untereinander, zu bewirken. Für ein gezieltes "Anwählen" einer Verbindung zum Lösen müßte dann bei sich zum Kopfende hin stufenweise vergrößernden Gewindedurchmessern ein Bolzen ausgesucht werden, dessen Außengewinde mit dem Innengewinde in Eingriff kommt, welches in dem auf der proximalen Seite der beabsichtigten Trennstelle gelegenen Schaftelements in Eingriff kommt. Er stützt sich dann mit dem Rand seines Endes auf dem Rand der Bohrung des distal benachbarten Elements ab und trennt die ausgewählte Verbindung.

Bei einem modularen System mit unterschiedlich gekrümmten Schaftelementen lassen sich individuell geformte Schäfte durch unterschiedliche relative Winkelpositionierung erzeugen, ohne daß die Lösbarkeit im vorstehendem Sinne irgendwie eingeschränkt wäre.

## Patentansprüche

1. Modulare Gelenkprothese mit einem Kopfteil und mindestens einem Schaftteil, die - vorzugsweise mittels eines Konusanschlusses - steckbar miteinander verbindbar sind, wobei ein Setzen bzw. Sichern der Steckverbindung mittels eines sich im wesentlichen koaxial zur Längsachse des Schaftteils durch eine beide Teile koaxial durchquerende Bohrung erstreckenden ersten Schraubmittels erfolgen kann, dadurch gekennzeichnet, daß die Gelenkprothese ein zweites Schraubmittel aufweist, und daß die im Kopfteil (3) oder einem dem distalen Schaftende proximal vorangehenden Schaftteil vorgesehene Bohrung (5) mit einem Gewinde versehen ist für das zweite Schraubmittel, welches zum Lösen der Steckverbindung mit dem nachfolgenden Schaftteil (2) einen äußeren Durchmesser (D₁) aufweist, der größer ist als der innere Durchmesser (D₂) der in dem nachfolgenden Schaftteil (2) angeordneten Bohrung (4).

2. Gelenkprothese nach Anspruch 1,
dadurch gekennzeichnet, daß das Verhältnis des größeren Durchmessers (D₁) zum kleineren Durchmesser (D₂) zwischen 1,5 und 2,5 beträgt.

3. Gelenkprothese nach Anspruch 1,
dadurch gekennzeichnet, daß sich das Gewinde in der im Kopfteil (3) bzw. in dem dem Kopfteil in Richtung nach distal vorangehenden Schaftteil angeordneten Bohrung (5) über deren gesamte Länge erstreckt.

4. Gelenkprothese nach Anspruch 1,
dadurch gekennzeichnet, daß die im nachfolgenden Schaftteil (2) vorgesehene Bohrung (4) nur im Bereich ihres proximalen Endes (6) mit einem Gewinde versehen ist.

5. Gelenkprothese nach einem der vorangehenden Ansprüche,
dadurch gekennzeichnet, daß das proximale Ende der Gewindebohrung (6) des nachfolgenden Schaftteils (2) eine Anfasung (11) aufweist.

6. Gelenkprothese nach Anspruch 5,
dadurch gekennzeichnet, daß die Anfasung (11) bezogen auf die Mittelachse der Gewindebohrung (6) einen Neigungswinkel von im wesentlichen 45° aufweist.

7. Gelenkprothese nach einem der vorangehenden Ansprüche,
dadurch gekennzeichnet, daß an der medialen Seite des Kopfteils (3) unterhalb des Gelenkanschluß-Konus (9) eine Auskehlung (9a) mit im wesentlichen bogenförmiger Begrenzung vorgesehen ist.

8. Bausatz zur Herstellung einer Gelenkprothese nach einem der vorangehenden Ansprüche,
gekennzeichnet durch eine Auswahl von Kopfteilen (3) unterschiedlicher Länge und/oder unterschiedlichen Durchmessers sowie eine Auswahl von Schaftteilen (2) unterschiedlicher Länge, unterschiedlichen Durchmessers und/oder unterschiedlicher Krümmung.

9. Bausatz nach Anspruch 8,
dadurch gekennzeichnet, daß für die Kopfteile (3) ein Durchmesser im Bereich von 12 bis 17 mm und für die Schaftteile (2) ein Längenbereich von 200 bis 320 mm und ein Durchmesserbereich von 10 bis 14 mm vorgesehen ist.

## Claims

1. A modular artificial hip joint having a head part and at least a shank part, which can preferably be inserted into one another by means of a cone connection, whereby a placing or securing of the insertable connection can result by means of a first screw means, extending essentially co-axially to the longitudinal axis of the shank part through a bore extending co-axially crossing through both parts, characterised in that the artificial hip joint has a second screw means, and that bore (5) provided in head-part (3) or a preceding shank part close to the distal shank end is provided with a thread for the second screw means, which has an outer diameter (D₁) which is greater than the inner diameter (D₂) of bore (4) arranged in following shank part (2) for releasing the insertable connection with the following shank part (2).

2. An artificial hip joint according to claim 1, characterised in that the ratio of the larger diameter (D₁) to the smaller diameter (D₂) amounts to between 1.5 and 2.5.

3. An artificial hip joint according to claim 1, characterised in that the thread arranged in the bore (5) in the head part (3), i.e. the head part in the direction of the distal-proximal preceding shank part, extends over its whole length.

4. An artificial hip joint according to claim 1, characterised in that the bore (4) provided in the following shank part (2) is only provided with a thread in the region of its proximal end (6).

5. An artificial hip joint according to any of the above claims, characterised in that the proximal end of the threaded bore (6) of the following shank part (2) has a chamfering (11).

6. An artificial hip joint according to claim 5, characterised in that the chamfering (11) related to the central axis of threaded bore (6) has an inclined angle of essentially 45°.

7. An artificial hip joint according to any of the above claims, characterised in that a groove (9a) having an essentially arc-shaped limitation is provided on the medial side of head part (3) below the joint-connecting cone (9).

8. A kit for producing an artificial hip joint according to any of the above claims, characterised by a selection of head parts (3) of differing length and of differing diameter, likewise a selection of shank parts (2) of differing length, differing diameter and/or differing curvature.

9. A kit according to claim 8, characterised in that a diameter in the range of 12 to 17mm is provided for the head parts (3), and a longitudinal range of 200 to 320 mm and a diameter of 10 to 14 mm is provided for the shank parts (2).

## Revendications

1. Prothèse articulaire modulaire comprenant une partie de tête et au moins une partie de fût, lesquelles sont susceptibles d'être reliées l'une à l'autre par enfichage, de préférence au moyen d'un raccordement à cône, dans lequel un serrage, ou un blocage de la liaison à enfichage peut avoir lieu au moyen d'un premier organe de vissage qui s'étend sensiblement coaxialement à l'axe longitudinal de la partie de fût à travers un perçage qui traverse coaxialement l'une des deux parties,
caractérisée en ce que la prothèse articulée comprend un deuxième organe de vissage, et en ce que le perçage (5) prévu dans la partie de tête (3), ou dans une partie du fût qui précède en direction proximale l'extrémité distale du fût, est doté d'un pas de vis pour le deuxième organe de vissage, celui-ci présentant, pour la libération de la liaison à enfichage avec la partie de fût (2) qui suit, un diamètre extérieur (D₁) supérieur au diamètre intérieur (D₂) du perçage (4) ménagé dans la partie de fût qui suit (2).

2. Prothèse articulaire selon la revendication 1,
caractérisée en ce que le rapport entre le grand diamètre (D₁) et le petit diamètre (D₂) est compris entre 1,5 et 2,5.

3. Prothèse articulaire selon la revendication 1,
caractérisée en ce que le pas de vis, dans le perçage (5) ménagé dans la partie de tête (3), ou dans une partie du fût qui précède en direction proximale l'extrémité distale du fût qui suit, s'étend sur la totalité de sa longueur.

4. Prothèse articulaire selon la revendication 1,
caractérisée en ce que le perçage (4) prévu dans la partie de fût qui suit (2) n'est doté d'un pas de vis que dans la zone de son extrémité proximale (6).

5. Prothèse articulaire selon l'une des revendications précédentes,
caractérisée en ce que l'extrémité proximale du perçage à pas de vis (6) de la partie de fût qui suit (2) présente un chanfrein (11).

6. Prothèse articulaire selon la revendication 5,
caractérisée en ce que, rapporté à l'axe médian du perçage à pas de vis (6), le chanfrein (11) présente un angle d'inclinaison sensiblement égal à 45°.

7. Prothèse articulaire selon l'une des revendications précédentes,
caractérisée en ce que, du côté médian de la partie de tête (3) au-dessous du cône (29) du raccordement d'articulation, il est prévu un évidement (9a) avec une délimitation sensiblement en forme d'arc.

8. Jeu de composants pour réaliser une prothèse articulaire selon l'une des revendications précédentes,
caractérisé par un assortiment de parties de tête (3) de longueurs différentes et/ou de diamètres différents, ainsi qu'un assortiment de parties de fût (2) de longueurs différentes, de diamètres différents, et/ou de courbures différentes.

9. Jeu de composants selon la revendication 8,
caractérisé en ce que l'on prévoit pour les parties de tête (3) un diamètre dans la plage de 12 à 17 mm, et pour les parties de fût (2) une plage en longueur de 100 à 320 mm et une plage en diamètre de 10 à 14 mm.
